# EUROPEAN PATENT APPLICATION

(11) **EP 4 644 545 A1**
(43) Date of publication of application: **05.11.2025**
(21) Application number: 23880892.7
(22) Date of filing: 24.11.2023
(51) Int. Cl.: C12N 15/11, C12N 15/63, C12N 9/00

(54) **METHOD FOR PRODUCING CIRCULAR RNA USING DUMBBELL STRUCTURE**

(30) Priority: 28.12.2022 KR 20220187737
(71) Applicant: Ribotech Inc., Seoul 02841 (KR)
(72) Inventor: OH, Na Ra, Goyang-si, Gyeonggi-do 10546 (KR); CHOI, Yeon Gil, Seoul 01914 (KR); KIM, Hyo Jeong, Seoul 02853 (KR)
(74) Representative: Potter Clarkson
(86) International application number: PCT/KR2023/019046
(87) International publication number: WO 2024/143924

(57) **Abstract**

The present disclosure relates to a circular RNA precursor having a dumbbell-shaped secondary structure, and the like. According to the present disclosure, the circular RNA precursor uses T4 RNA ligase 2 (Rnl2), but does not require a DNA splint in preparing circular RNA, and furthermore, it is possible to prepare circular RNA with greater efficiency than using the DNA splint. In addition, in preparation of circular RNA using a ribozyme, the ribozyme needs to be finally removed, but in the present disclosure, an additional RNA removal step is not required. In addition, the circular RNA precursor of the present disclosure may be prepared as circular RNA containing a protein-encoding gene as GOI, and thus can be used as a gene therapy for the purpose of protein expression.

## Description

### Technical Field

The present disclosure relates to a circular RNA precursor with a dumbbell structure capable of preparing circular RNA using T4 RNA ligase 2 without a DNA splint, a method for preparing circular RNA using the same, and the like.

### Background Art

Since coronavirus disease 19 (COVID-19), an infectious disease of SARS-CoV-2, first identified in Wuhan, China, in 2019, has become a global pandemic, the development of vaccine treatments using messenger RNA (mRNA) has been more actively conducted. mRNA has a limitation of being easily degraded *in vivo* and having a relatively short half-life. To overcome such a limitation, research has been conducted on improving stability by adding a poly(A)tail to mRNA.

Meanwhile, circular RNA (circRNA) is a single-stranded transcript linked by covalent bonds, and is very stable *in vivo* due to its structural characteristic of having no 5' and 3' ends. Accordingly, many studies have been conducted to use circRNA to overcome the low stability limit of mRNA, and in the same context, US 10,953,033 discloses circRNA for the purpose of gene expression *in vivo* based on the structural characteristics of circRNA.

In eukaryotes, circRNA is produced through back-splicing from mRNA, but it has been known that T4 RNA ligase 1 (Rnl1), T4 RNA ligase 2 (Rnl2), or Group 1 intronic ribozyme may be used for preparing circRNA *in vitro.* The size of circRNA for the purpose of protein expression is relatively large, so that circRNA preparation efficiency is low, and there is a problem in the isolation and purification of circRNA.

T4 RNA ligase 2 (Rnl2) is an enzyme for linking a 3'-end and a 5'-end of double-stranded RNA or double-stranded RNA/DNA hybrid, and it is general to use DNA splints capable of binding complementarily to both ends of single-stranded RNA when preparing circRNA.

The present inventors conducted intensive research on a method for improving the preparation efficiency and reducing a problem of separation and purification in the preparation of circRNA for the purpose of protein expression and then completed the present disclosure.

### Disclosure of the Invention

### Technical Goals

The present disclosure has been made in an effort to provide a circRNA precursor capable of being circularized without a DNA splint in preparation of circRNA using T4 RNA ligase 2 and a method for preparing circRNA efficiently using the same.

However, technical goals to be achieved are not limited to those described above, and other goals not mentioned above are clearly understood by one of ordinary skill in the art from the following description.

### Technical Solutions

An embodiment of the present disclosure provides a circular RNA precursor circularized by Rnl2 without a DNA splint.

The circular RNA precursor of the present disclosure has the following structure:
5'- 3'DumbBell Sense sequence (3'DB SS) - GOI (gene of interest) - 3'DumbBell Antisense sequence (3'DB AS) - 3'.

In addition, the circular RNA precursor of the present disclosure may have the following structure:
5'- 5'DumbBell Antisense sequence (5'DB AS) - 5'DumbBell Sense sequence (5'DB SS) -3'DumbBell Sense sequence (3'DB SS) - GOI - 3'DumbBell Antisense sequence (3'DB AS) - 3'.

As an embodiment of the present disclosure, the 3'DB AS region consists of a reverse complementary sequence to the 3'DB SS region, the 3'DB AS region and the 3'DB SS region form base pairs, and a region where the 3'DB AS region and the 3'DB SS region form the base pairs is referred to as 3' dumbbell (3'DB).

As another embodiment of the present disclosure, the 3'DB SS region may have a length of 10 to 29 nt.

As another embodiment of the present disclosure, the 5'DB AS region consists of a reverse complementary sequence to the 5'DB SS region, the 5'DB AS region and the 5'DB SS region form base pairs, and a region where the 5'DB AS region and the 5'DB SS region form the base pairs is referred to as 5' dumbbell (5'DB).

As another embodiment of the present disclosure, the 5'DB SS region may have a length of 3 to 15 nt.

As another embodiment of the present disclosure, the total length of the 3'DB and 5'DB may be 30 bp or less.

As another embodiment of the present disclosure, the length of the GOI region may be 500 to 5500 nt.

As another embodiment of the present disclosure, the GOI region may further include an IRES for protein expression.

Another embodiment of the present disclosure provides an expression vector capable of expressing the circular RNA precursor.

In an embodiment of the present disclosure, the expression vector may include a gene encoding the circular RNA precursor and a promoter linked to enable *in vitro* transcription (IVT), a process of synthesizing RNA *in vitro* using RNA polymerase, NTPs, and various cofactors using DNA as a template.

Yet another embodiment of the present disclosure provides a method for preparing circular RNA with high efficiency using the circular RNA precursor and Rnl2. The method includes the following steps:
(1) inducing *in vitro* transcription of the expression vector of claim 6 under a condition of GTP : GMP = 1 : 5 to 10;
(2) treating DNase I *in vitro* after the transcription induction step is completed; and
(3) inducing ligation by adding Rnl2 *in vitro.*

As an embodiment of the present disclosure, the method may further include treating RppH *in vitro* after performing step 2.

As another embodiment of the present disclosure, the method may further include a heating step immediately before step 3, and the heating step includes heating *in vitro at* 80°C for 3 minutes and cooling at 6°C per minute until reaching 26°C.

As another embodiment of the present disclosure, step 3 may be performed at 25°C for 1 h or more.

As another embodiment of the present disclosure, the method may further include (4) treating RNase R *in vitro* after step 3.

As another embodiment of the present disclosure, the method may further include treating poly (A) polymerase *in vitro* immediately before step 4.

### Effects

According to the present disclosure, the circular RNA precursor uses T4 RNA ligase 2 (Rnl2), but does not require a DNA splint in preparing circular RNA, and furthermore, it is possible to prepare circular RNA with greater efficiency than using the DNA splint. In addition, in preparation of circular RNA using a ribozyme, the ribozyme needs to be finally removed, but in the present disclosure, an additional RNA removal step is not required. In addition, the circular RNA precursor of the present disclosure may be prepared as circular RNA containing a protein-encoding gene as GOI, and thus may be used as a gene therapy for the purpose of protein expression.

### Brief Description of Drawings

FIG. 1 is a diagram briefly illustrating expression of a dumbbell circular RNA precursor of the present disclosure and preparation and purification of circular RNA by Rnl2.

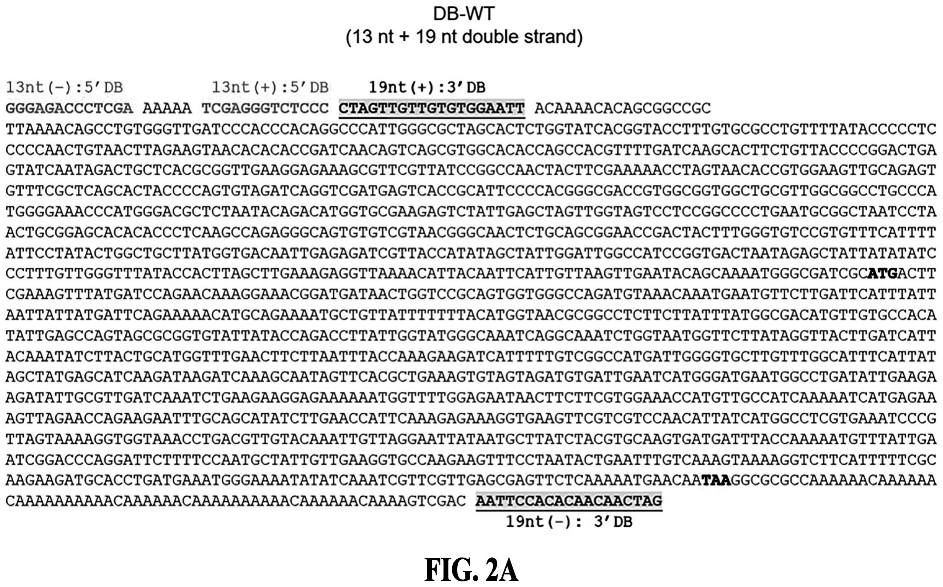

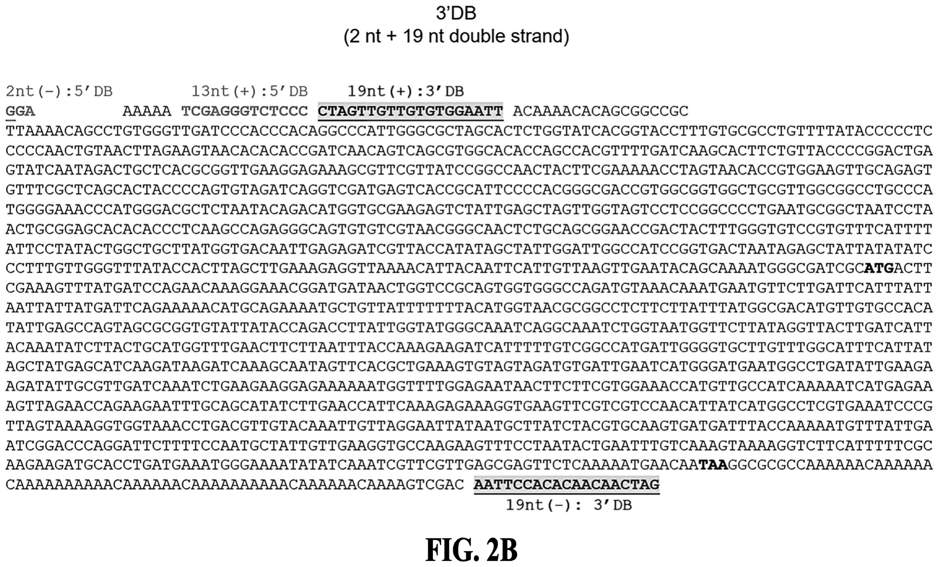

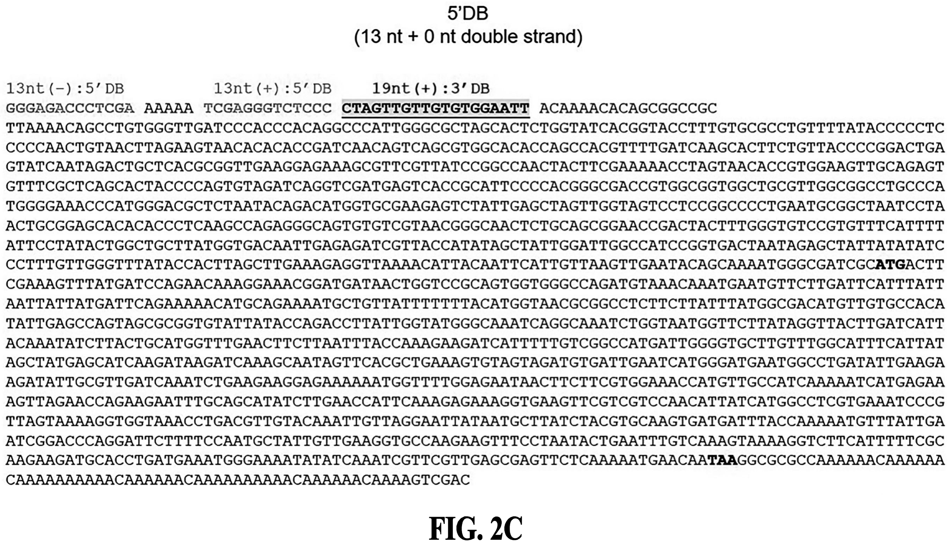

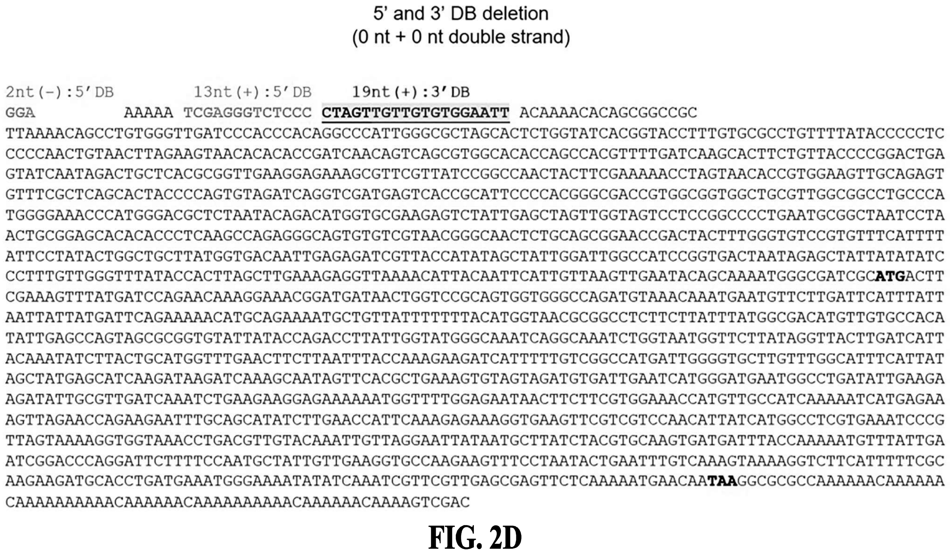

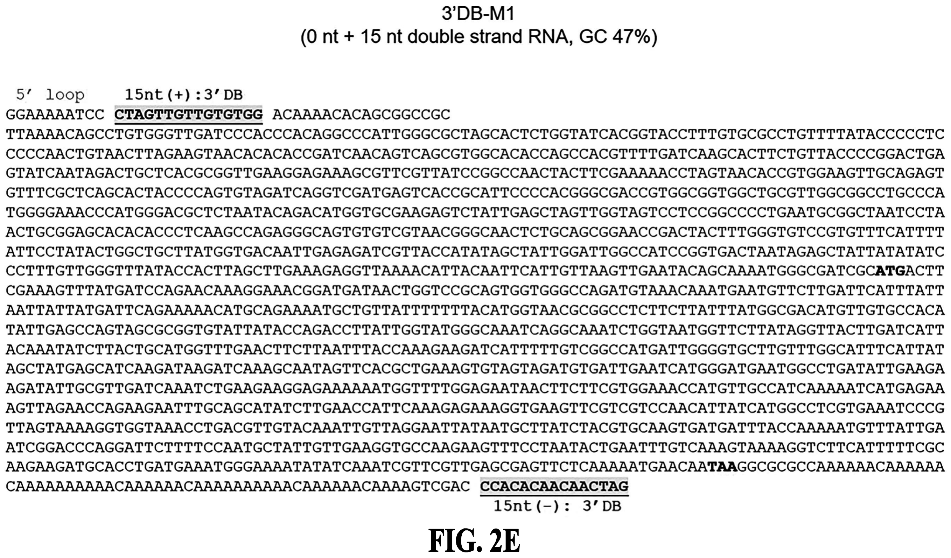

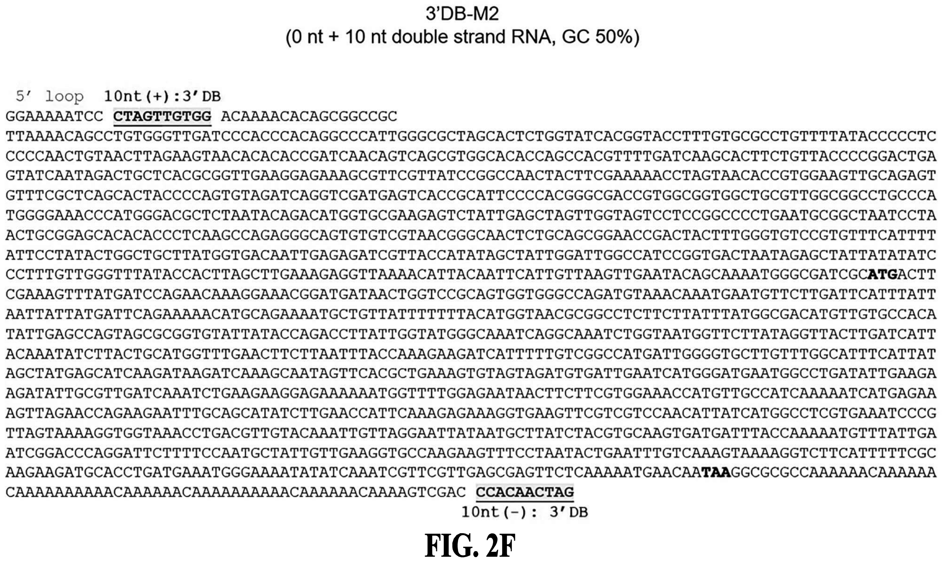

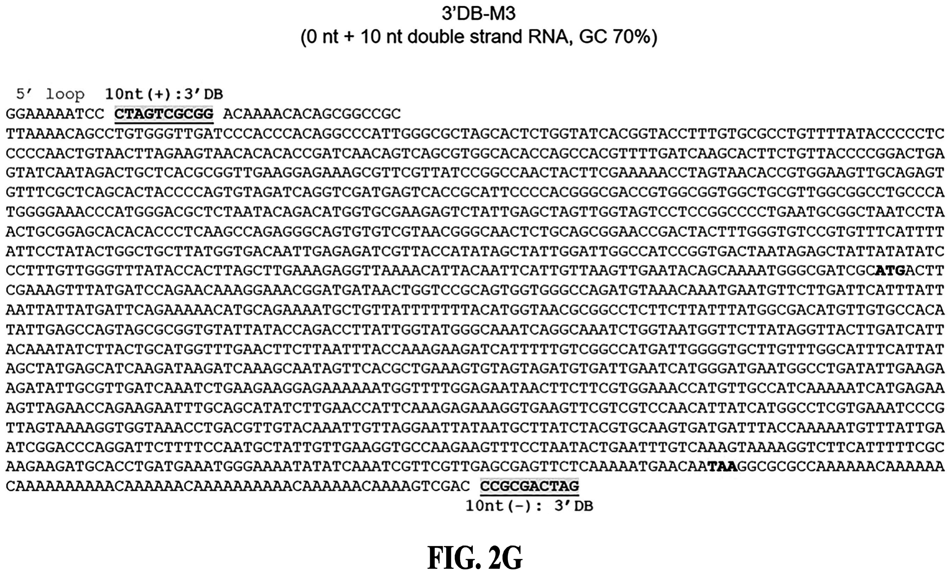

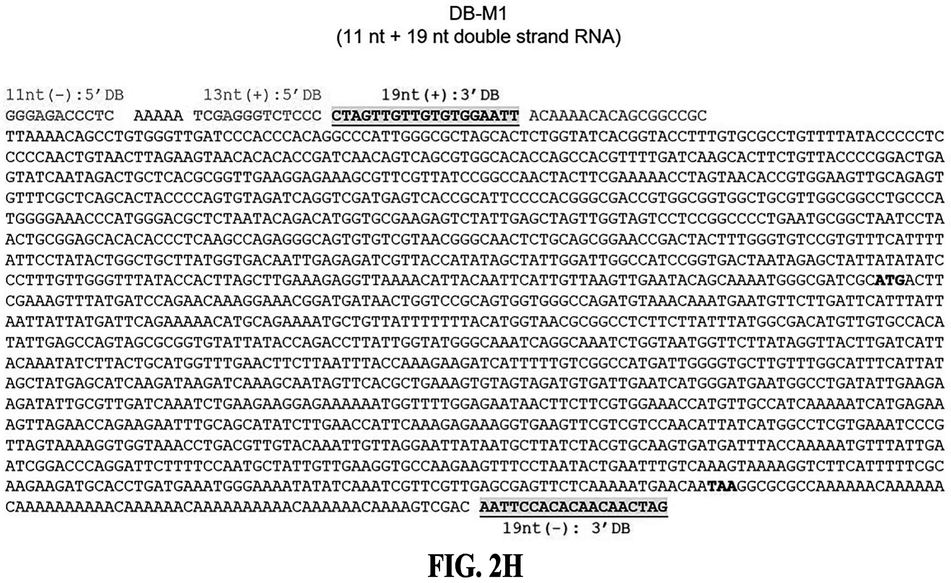

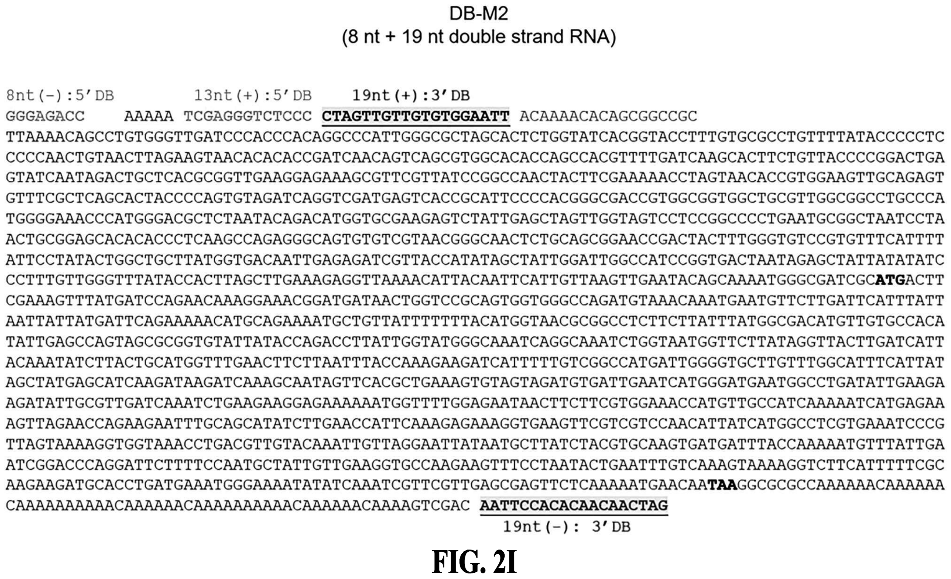

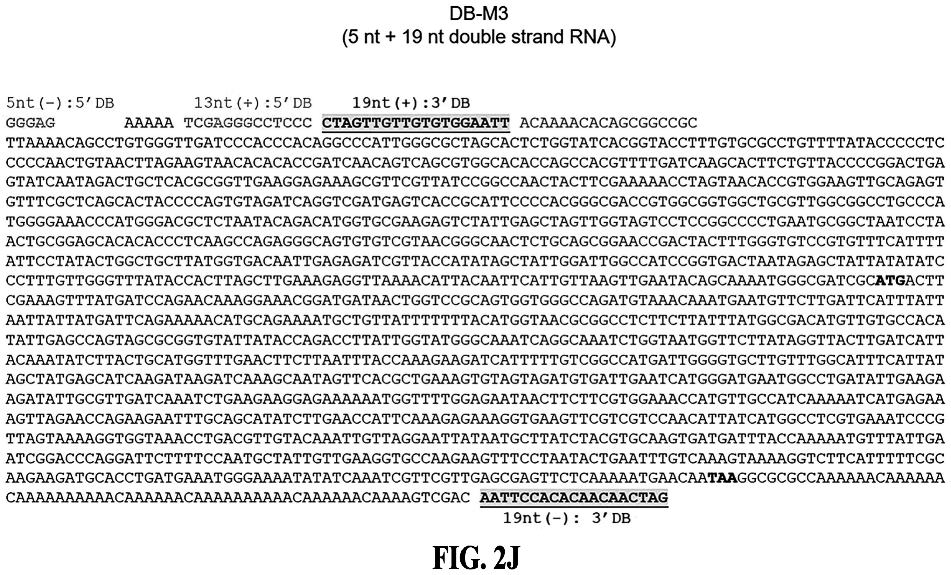

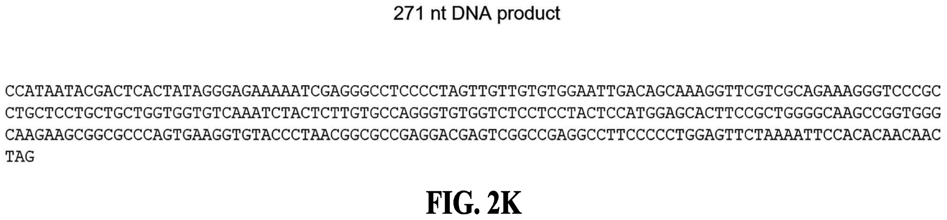

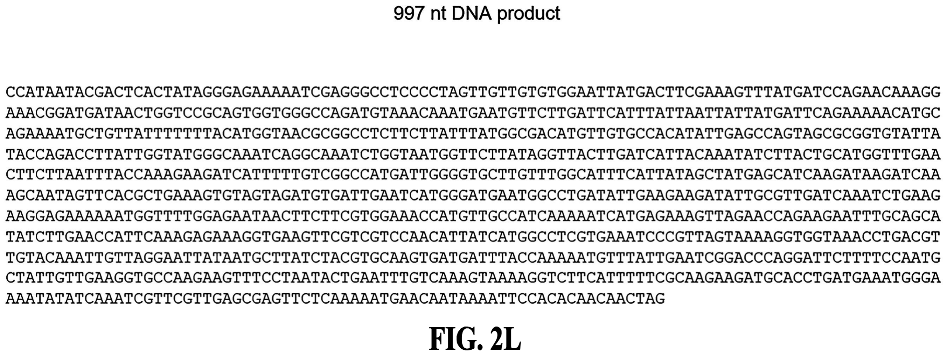

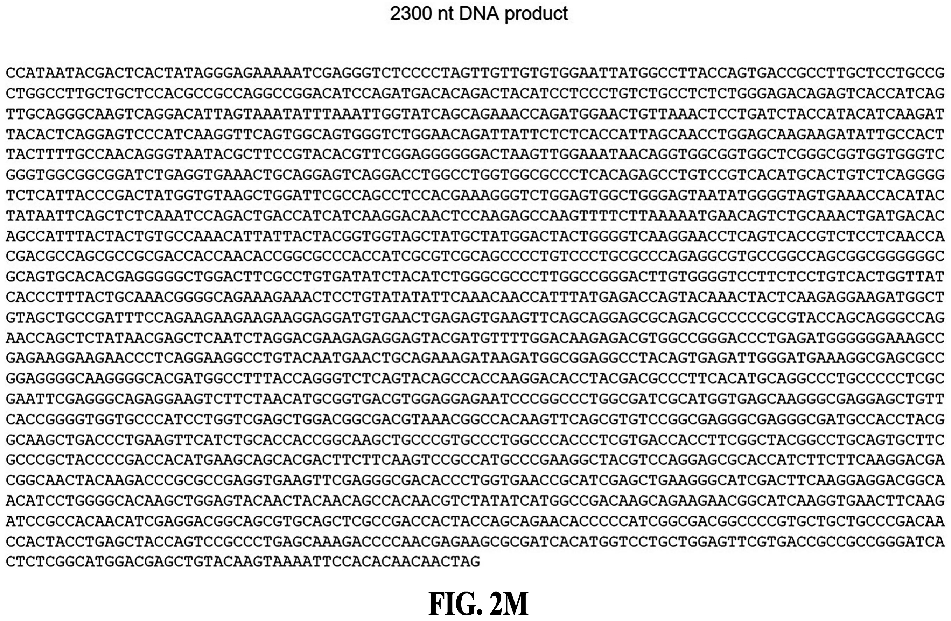

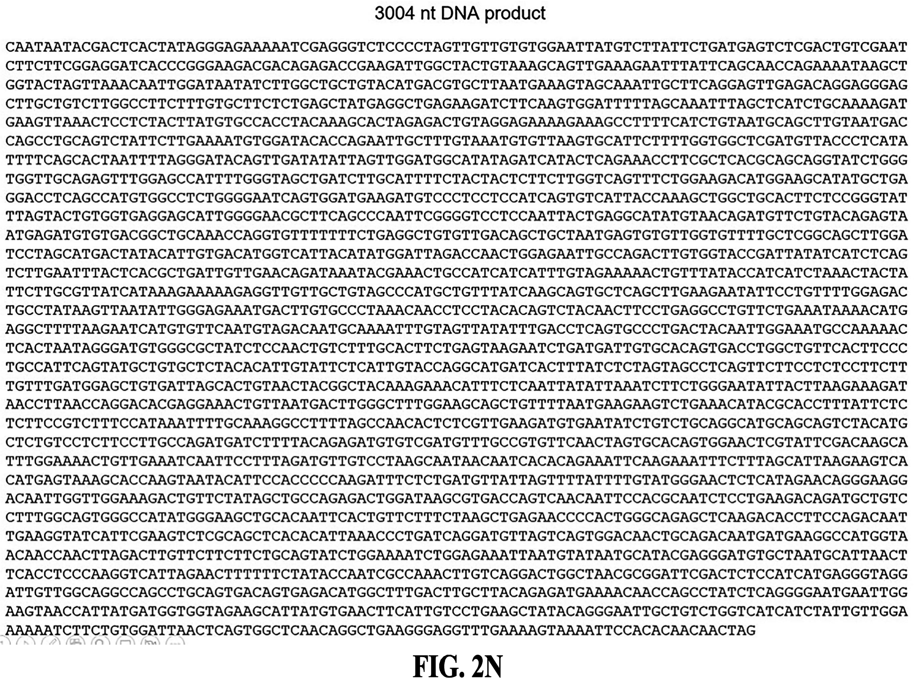

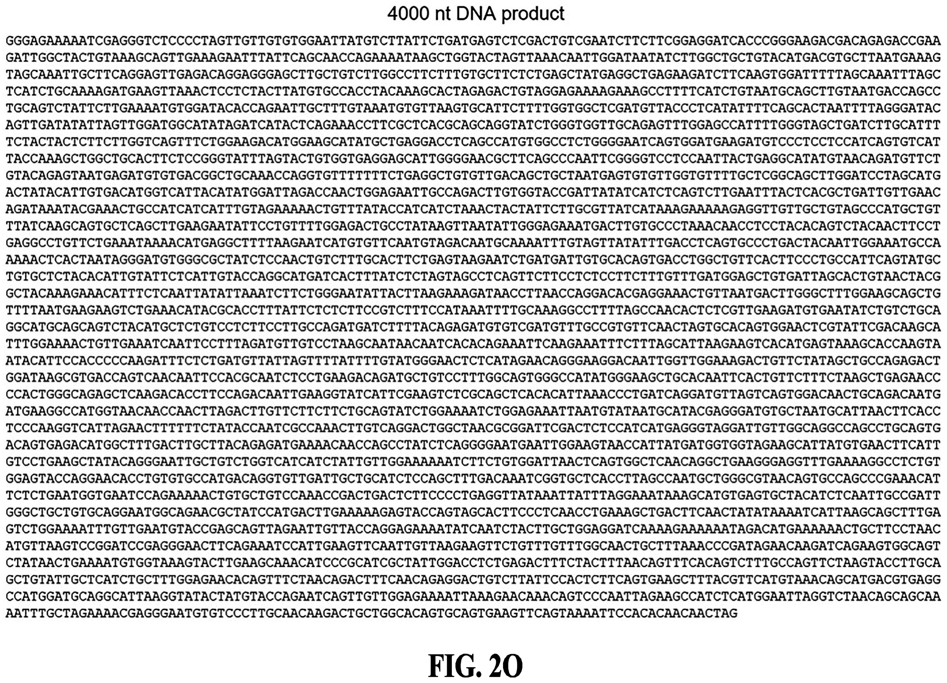

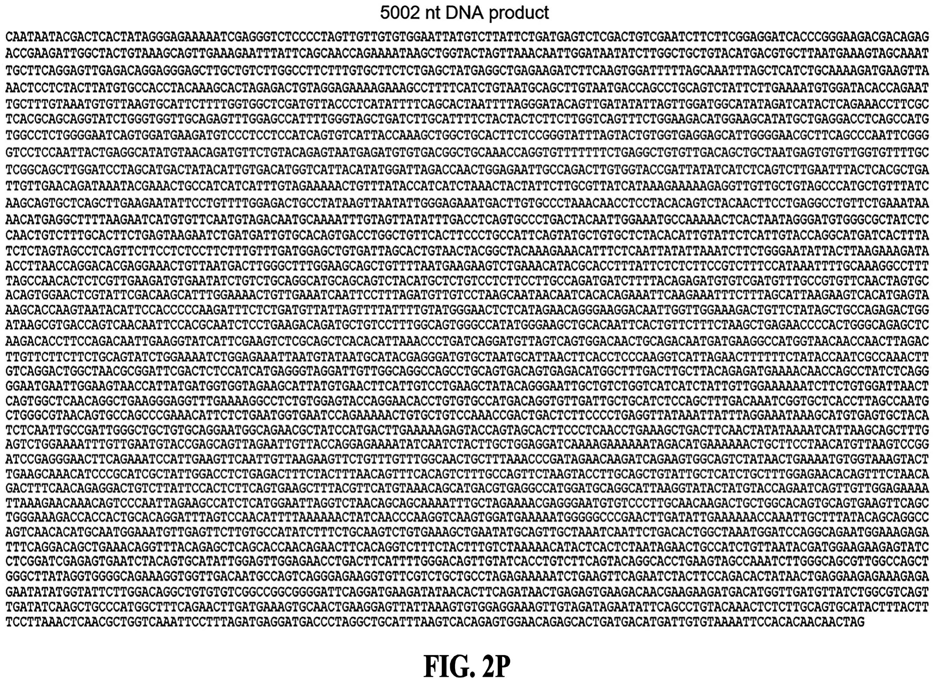

FIG. 2 is a diagram illustrating DNA sequences and structures expressing dumbbell circular RNA precursors with various structures. FIG. 2A illustrates a sequence of a wild type DB gene, FIG. 2B illustrates a sequence of a 3'DB gene, FIG. 2C illustrates a sequence of a 5'DB gene, FIG. 2D illustrates a sequence of a 5'3' DB deletion gene, FIG. 2E illustrates a sequence of a 3'DB-M1 gene, FIG. 2F illustrates a sequence of a 3'DB-M2 gene, FIG. 2G illustrates a sequence of a 3'DB-M3 gene, FIG. 2H illustrates a sequence of a DB-M1 gene, FIG. 2I illustrates a sequence of a DB-M2 gene, and FIG. 2J illustrates a sequence of a DB-M3 gene. FIG. 2K illustrates a DB-M3 gene of 271 nt, FIG. 2L illustrates a DB-M3 gene of 997 nt, FIG. 2M illustrates a DB-M3 gene of 2300 nt, FIG. 2N illustrates a DB-M3 gene of 3004 nt, FIG. 2O illustrates a DB-M3 gene of 4000 nt, and FIG. 2P illustrates a DB-M3 gene of 5002 nt.
FIG. 3 illustrates results of confirming that a 3' dumbbell region is essential for ligation by Rnl2 in a circular RNA precursor with a dumbbell structure.
FIG. 4 illustrates results of confirming ligation efficiency by Rnl2 of circular RNA mutant precursors 3'DB-M designed by varying a length and a GC ratio of a 3'DB region in order to confirm an effect of the length of the 3'DB region on the preparation efficiency of circular RNA.
FIG. 4A illustrates a design diagram of 3'DB and 3'DB mutant precursors 3'DB-M, and FIG. 4B illustrates a result of confirming ligation efficiency after inducing ligation of each circular RNA precursor by Rnl2.
FIG. 5A illustrates a design diagram of circular RNA mutant precursors DB-M designed by varying a length of a 5'DB region in order to confirm an effect of the length of the 5'DB region on the preparation efficiency of circular RNA and FIG. 5B illustrates a result of confirming ligation efficiency of each mutant precursor by Rnl2.
FIG. 6 illustrates results of confirming the lengths of a gene capable of being inserted into circular RNA, in which FIG. 6A illustrates a result of electrophoresis on a formaldehyde agarose gel, and FIG. 6B illustrates a result of electrophoresis on an EX 2% agarose gel.
FIG. 7 illustrates a result of confirming the ligation efficiency of a circular RNA precursor according to a ratio of GTP and GMP when performing IVT.
FIG. 8 illustrates a result of confirming the ligation efficiency of a circular RNA precursor according to heat treatment temperature and time and cooling conditions of a heating step before Rnl2 treatment.
FIG. 9 illustrates a result of confirming the ligation efficiency of a circular RNA precursor according to ligation temperature and time conditions.
FIG. 10 illustrates a result of confirming the ligation efficiency of a circular RNA precursor depending on the presence or absence of an RppH treatment step before ligation induction.
FIG. 11 illustrates confirming a result of IVT and circularization induction of DB-WT under various conditions.
FIG. 12 illustrates confirming a result of IVT and circularization induction of 3'DB under various conditions.
FIG. 13 is a diagram of comparing and confirming ligation efficiency of preparation of circular RNA using a circular RNA precursor with a dumbbell structure and preparation of circular RNA using a DNA splint.

### Best Mode for Carrying Out the Invention

The present inventors developed a circular RNA precursor with a dumbbell structure that may be circularized by T4 RNA ligase 2 without a DNA splint in the preparation of circular RNA for the purpose of protein expression *in vivo* as a gene therapy and developed and completed various mutant precursors to improve the ligation efficiency of the circular RNA precursor.

T4 RNA ligase 2 (Rnl2) may link a 3'-end and a 5'-end of double-stranded RNA. Accordingly, a self-hybridizing circular RNA precursor capable of forming a dumbbell structure by complementarily binding to an RNA interior at the 5' and 3' ends was designed and prepared, and it was confirmed that the RNA precursor may be circularized by Rnl2 without a DNA splint.

The "circular RNA precursor" provided by the present disclosure has both ends self-hybridizing with its interior to form a dumbbell-like secondary structure, and is used interchangeably with the terms "circular RNA precursor with a dumbbell structure," "self-hybridization circular RNA precursor," and "dumbbell circular RNA precursor".

As used herein, a double strand RNA (dsRNA) region formed by complementary binding of the 5' end of the circular RNA precursor to the interior of RNA is referred to as a "5'DB region", and a dsRNA region formed by complementary binding of the 3' end to the interior of RNA is referred to as a "3'DB region".

As used herein, when the 5'DB region and the 3'DB region are not distinguished, but when a region forming dsRNA is indicated, the region is described as a "DB region".

Meanwhile, the secondary structure of RNA is variable depending on the binding force between bases and environmental conditions. Accordingly, as used herein, the DB region refers to a region where three or more nucleotides complementarily bind to each other, that is, a dsRNA region of 3 bp or more.

As used herein, in the DB region, it is desirable that three or more nucleotides complementarily bind to each other in sequence to form base pairs, but as the length of the DB region increases, a case of including nucleotides that do not form one or two base pairs within the range of maintaining the binding force is not excluded.

As used herein, the RNA sequences constituting the 5'DB region are referred to as a "5'DB sense sequence (5'DB SS)" and a "5'DB antisense sequence (5'DB AS)", respectively, and the RNA sequences constituting the 3'DB region are referred to as a "3'DB sense sequence (3'DB SS)" and a "3'DB antisense sequence (3'DB AS)", respectively.

The circular RNA precursor of the present disclosure may additionally include random nucleotides of 1 to 19 nt in length to form a loop between 5'DB SS and 5'DB AS.

The circular RNA precursor of the present disclosure includes a gene of interest (GOI) between 3'DB SS and 3'DB AS, and the GOI may further include an IRES for protein expression and may further include a UTR region to improve translation efficiency.

Meanwhile, an effect of the 5'DB region and the 3'DB region on ligation efficiency was confirmed in the circular RNA precursor that forms a dumbbell structure by self-hybridization of the present disclosure. Specifically, the previously designed circular RNA precursor of the dumbbell structure has a 5' DB region of 13 bp long and a 3' DB region of 19 bp long, and as used herein, the circular RNA precursor was named "DB-WT". Based on the DB-WT, a 3' dumbbell circular RNA precursor without a 5'DB region (hereinafter referred to as "3'DB"), a 5' dumbbell circular RNA precursor without a 3'DB region (hereinafter referred to as "5'DB"), and a 5' and 3' dumbbell deletion precursor without forming a dumbbell structure (hereinafter referred to as "5'3'DB deletion") were designed, and ligation was induced by Rnl2.As a result, it was confirmed that the 3'DB region is essential for ligation by Rnl2, but the 5'DB region is not essential.

Accordingly, the present inventors provide a circular RNA precursor including only a 3'DB region without a 5'DB region. Even in the case, the 3' end of the circular RNA precursor of the present disclosure self-hybridizes with its interior, and as used herein, the terms "circular RNA precursor with a dumbbell structure" and "self-hybridization circular RNA precursor" are used as the meaning of including circular RNA precursors including only the 3'DB region.

Within cells, long dsRNA is recognized as a foreign substance and activates a STING pathway to induce cellular immune responses. Accordingly, circular RNA that induces cellular immune responses is not suitable for use as a gene therapy. According to past studies, it is known that dsRNA of 40 bp or longer is recognized by TLR3 to induce immune responses (Schlee et al, Nature Review, https://www.nature.com/articles/nri.2016.78). In addition, it is known that dsRNA of 30 bp or longer activates phosphatase, PKR to induce immune responses, whereas dsRNA of 25 bp or less does not activate PKR (Lemaire et al, 2009, JMB, https://www.ncbi.nlm.nih.gov/pmc/articles/PMC2570377/, Manche et al, 1992, MCB, https://pubmed.ncbi.nlm.nih.gov/1357546/). DB-WT includes a DB region with a total length of 32 bp, including a 5'DB region of 13 bp and a 3'DB region of 19 bp. Accordingly, the present inventors searched for a circular RNA precursor structure having suitable ligation efficiency while reducing the length of the DB region so as not to induce immune responses within the cells.

As a specific experimental example, based on 3'DB, three types of circular RNA mutant precursors (hereinafter referred to as "3'DB-M") were prepared by varying a length and a GC content of the 3'DB region. The three types of 3'DB-M were designed as shown in Table 3, and as a result of confirming the ligation efficiency by Rnl2 by comparing the three types of 3'DB-M with 3'DB, it was confirmed that the ligation efficiency decreased as the length of the 3'DB region became shorter, and the effect of the GC content of the 3'DB region on the ligation efficiency was insignificant. Accordingly, in the range without inducing the cellular immune responses, the longer the 3'DB region was, the higher the ligation efficiency was.

Next, in the DB-WT, the length of the 3'DB region was set to 19 bp and the length of the 5'DB region was reduced to prepare three types of circular RNA mutant precursors (hereinafter referred to as "DB-M"). As a result, it was confirmed that there was no significant difference in ligation efficiency when the length of the 5'DB region was 5 bp compared to when the length was 13 bp. Accordingly, in the circular RNA precursor capable of producing circular RNA with high efficiency while minimizing the possibility of inducing cellular immune responses, there are provided a 5'DB region of 5 bp in length and a 3'DB region of 19 bp in length.

The circular RNA structure with the dumbbell structure of the present disclosure may produce circular RNA including genes with a length of about 6000 nt or more, but in terms of preparing efficiency, the length of the gene included in the circular RNA structure is desirably 200 to 5,500 nt, and more desirably 500 to 3,500 nt.

In addition, the present inventors developed a method for increasing the circular RNA production efficiency when preparing circular RNA using the circular RNA precursor with the dumbbell structure of the present disclosure.

Accordingly, in a specific experiment, it was confirmed that IVT was performed by adding GMP to induce ligation by T4 RNA ligase 2 without an RppH treatment step, and the circular RNA production yield was excellent at a ratio of GTP : GMP = 1 : 1 to 10. Therefore, the present inventors are able to provide a method of producing circular RNA, excluding the RppH treatment process, by performing IVT under the GTP : GMP ratio.

In addition, the present disclosure induced ligation by Rnl2 by forming a secondary structure of a circular RNA precursor, and induced desired ligation by adding a heating step before Rnl2 treatment. At this time, it was confirmed that circular RNA production efficiency may be improved by adding a heating step of heat-treating at 80°C for 3 minutes and cooling to 26°C by 6°C per minute.

Furthermore, it was confirmed that the ligation temperature condition was suitably 25°C, the ligation reaction time was sufficient for 1 hour, and the yield of circular RNA could be further increased by adding the RppH treatment step. However, the increase in circular RNA production yield by RppH treatment may be obtained more effectively from 3'DB than DB-WT, and thus, it was confirmed that when the stability of the 5'DB region increases, RppH was not easily accessible to the 5' end due to the secondary structure of the circular RNA precursor. Even after circular RNA purification, a result of confirming that DB-WT shows darker noise bands than 3'DB is caused by difficulty in access of the enzyme to the end thereof due to the secondary structure of an uncircularized precursor when the stability of the 5'DB region is high.

Meanwhile, the present inventors are able to secure higher circular RNA preparation efficiency than splint ligation when using the circular RNA precursor with the dumbbell structure of the present disclosure, and thus, the circular RNA precursor of the present disclosure is expected to be useful in preparing circular RNA using Rnl2.

In the present disclosure, the circular RNA precursor includes natural ribonucleotides of adenosine, guanosine, cytosine, and uracil, and may also include nonnatural ribonucleotides.

The present disclosure provides a cassette, a gene delivery system, and a vector for expressing the above-described circular RNA precursor with the dumbbell structure, and as used herein, in describing the circular RNA precursor and the gene encoding the same, it is obvious to those skilled in the art that thymine in the gene corresponds to uracil in the circular RNA precursor.

### Mode for Carrying Out the Invention

Hereinafter, Examples will be described in detail with reference to the accompanying drawings. However, since various modifications may be made to Examples, the scope of the present disclosure is not limited or restricted by these Examples. It should be understood that all modifications, equivalents and substitutes for Examples are included in the scope of the present disclosure.

The terms used in Examples are used for the purpose of description only, and should not be construed to be limited. The singular expression includes the plural expression unless the context clearly dictates otherwise. In the present disclosure, it should be understood that the term "including" or "having" indicates that a feature, a number, a step, an operation, a component, a part or the combination thereof described in the specification is present, but does not exclude a possibility of presence or addition of one or more other features, numbers, steps, operations, components, parts or combinations thereof, in advance.

Unless otherwise contrarily defined, all terms used herein including technological or scientific terms have the same meanings as those generally understood by a person with ordinary skill in the art to which Examples pertain. Terms which are defined in a generally used dictionary should be interpreted to have the same meaning as the meaning in the context of the related art, and are not interpreted as ideal or excessively formal meanings unless otherwise defined in the present disclosure.

In addition, in the description with reference to the accompanying drawings, like components designate like reference numerals regardless of reference numerals and a duplicated description thereof will be omitted. In describing the Examples, a detailed description of related known technologies will be omitted if it is determined that they unnecessarily make the gist of the Examples unclear.

### [Experimental Method]

### 1. Preparation of DNA template for expressing circular RNA precursor with dumbbell structure

### 1-1. Wild type dumbbell circular RNA precursor (DB-WT)

To produce pUC-Dumbbell (DB), a T7 RNA polymerase promoter, a dumbbell, an IRES, and an RLuc poly A sequence were first synthesized (Integrated DNA Technologies) and cloned into a pUCIDT vector. Subsequently, pUC-DB was linearized by treating a SpeI restriction enzyme. A DB gene included a 13 nt-long 5'DumbBell Antisense sequence (5'DB AS) and 5'DumbBell Sense sequence (5'DB SS) and a 19 nt-long 3'DumbBell Sense sequence (3'DB SS) at a 5' end and included a 19 nt-long 3'DumbBell Antisense sequence (3'DB AS) at a 3' end (FIG. 2A).

### 1-2. 3' dumbbell circular RNA precursor (3'DB)

PCR was performed by using pUC-DB as a template and using primers 5'-GCGCTGTTTAAACTAATACGACTCACTATAGGAAAAAATCGAGGGTCTCCCCT AGTTG-3' (sense: SEQ ID NO. 1) and 5'-GCGCTGCGATCGCCCATTTTGCTGTATTCAA-3' (antisense: SEQ ID NO. 2) to ligate a PmeI/AsisI fragment of pUC-DB in an amplification product. The underlined sequences in each of the sense and antisense primers refers to PmeI and AsiSI recognition sites, respectively. pUC-3'DB was prepared by performing PCR using an Advantage-HF2 PCR kit (Clonetech).

Then, pUC-3'DB was cleaved with SpeI and then linearized. The 3'DB gene included a 19 nt-long 3'DB SS together with a 2 nt-long 5'DB SS and 5'DB AS at the 5' end and a 19 nt-long 3'DB AS at the 3' end (FIG. 2B).

### 1-3. 5' dumbbell circular RNA precursor (5'DB)

pUC-DB was cleaved with Sall to obtain a 5' dumbbell DNA fragment. The 5'DB gene included only a 13 nt-long 5'DB SS and 5'DB AS at the 5' end (FIG. 2C).

### 1-4. 5' and 3' dumbbell deletion precursor (5'3'DB deletion)

A 5' and 3' dumbbell deletion DNA fragment was obtained by cleaving pUC-3'DB with SalI. A 5' and 3' DB deletion gene did not include dumbbell-forming sequences at both ends (FIG. 2D).

### 1-5. 3' dumbbell circular RNA mutant precursor (3'DB-M)

To prepare 3'DB mutants, the lengths of a 5' loop and a 3'DB region were shortened, and the GC ratios in the 3'DB region were changed to 47%, 50%, and 70%. The 5' loop refers to a region without forming base pairs between 5'DB SS and 5'DB AS.

3'DB mutant 1 (3'DB-M1) was obtained as a PCR amplification product by using pUC-3'DB as a template and primers of 5- CTTTGTT TAAACTAATACGACTCACTATAGGAAAAATCCCTAGTTGTTGTGTGGACAAAA CACAGCGGCCGCTTAAAACAGC-3' (sense: SEQ ID NO. 3) and 5'-TGGACTAGTTGTTGTGTGG GTCGACTTTTGTTTTTTGTTTTTTTTTTGTTT-3' (antisense: SEQ ID NO. 4) (FIG. 2E). The underlined sequences in the primers were sites recognized by SpeI.

3'DB mutant 2 (3'DB-M2) was obtained as a PCR amplification product by using pUC-3'DB as a template and primers of 5'- CTTTGTTTAAACTAATACGACTCACTA TAGGAAAAATCCCTAGTTGTGGACAAAACACAGCGGCCGCTTAAAACAGC-3' (sense: SEQ ID NO. 5) and 5'-TGGACTAGTTGTGGGTCGACTTTTGTTTTTTGTTTTTTTTTTGTT-3' (antisense: SEQ ID NO. 6) (FIG. 2F). The underlined sequences in the primers were sites recognized by SpeI.

3'DB mutant 3 (3'DB-M3) was obtained as a PCR amplification product by using pUC-3'DB as a template and primers of 5'- CTTTGTTTAAACTAATACGACTCAC TATAGGAAAAATCCCTAGTCGCGGACAAAACACAGCGGCCGCTTAAAACAG C-3' (sense: SEQ ID NO. 7) and 5'-TGGACTAGTCGCGGGTCGACTTTTGTTTTTTGTTTTTTTTTTGTTT-3' (antisense: SEQ ID NO. 8) (FIG. 2G). The underlined sequences in the primers were sites recognized by SpeI.

### 1-6. Dumbbell circular RNA mutant precursors (DB-M)

DB mutants were prepared by reducing the 13 bp-long 5'DB region in the DB gene from 11 bp to 2 bp.

DB mutant 1 (DB-M1) was obtained as a PCR amplification product by using pUC-3'DB as a template and primers of 5'-TAATACGACTCACTATAGGGAGACCCTC AAAAATCGAGGGTCTCCCCTAGTTGTTG-3' (sense: SEQ ID NO. 9) and 5'-CTAGTTGTTGTGTGG AATTGTCGACTTTTGT-3' (antisense: SEQ ID NO. 10) (FIG. 2H). DB mutant 1 had a 5'DB region length of 11 bp.

DB mutant 2 (DB-M2) was obtained as a PCR amplification product by using pUC-3'DB as a template and primers of 5'- TAATACGACTCACTATAGGGAGACCAA AAATCGAGGGTCTCCCCTAGTTGTTG-3' (sense: SEQ ID NO. 11) and 5'-CTAGTTGTTGTGTGG AATTGTCGACTTTTGT-3' (antisense: SEQ ID NO. 12) (FIG. 2I). DB mutant 2 had a 5'DB region length of 8 bp.

DB mutant 3 (DB-M3) was obtained as a PCR amplification product by using pUC-3'DB as a template and primers of 5'- TAATACGACTCACTATAGGGAGAAAAA TCGAGGGCCTCCCCTAGTTGTTG -3' (sense: SEQ ID NO. 13) and 5'-CTAGTTGTTGTGTGG AATTGTCGACTTTTGT-3' (antisense: SEQ ID NO. 14) (FIG. 2J). DB mutant 3 had a 5'DB region length of 5 bp.

### 1-7. Circular RNA precursors with different GOI lengths

Circular RNA precursors were prepared by varying genes inserted into the GOI. A DNA fragment encoding each circular RNA precursor was amplified through PCR using primers listed in Table 1 below, and each amplified DNA product was shown in FIGS. 2K to 2P. The total length of the 5' and 3' DB regions in each circular RNA precursor was 24 bp.

**[Table 1]**

| Length of circular RNA precursor | Primer | SEQ ID NO: |
|---|---|---|
| 271 nt | 5'- CCATAATACGACTCACTATAGGGAGAAAAAT CGAGGGCCTCCCCTAGTTGTTGTGTGGAAT TATGGACAGCAAAGGTTCGTCGC-3' | 15 |
| | 5′- CTAGTTGTTGTGTGGAATTTTAGAACTCCAG GGGGAAGGCC-3′ (antisense) | 16 |
| 997nt | 5'- CCATAATACGACTCACTATAGGGAGAAAAAT CGAGGGCCTCCC CTAGTTGTTGTGTGGAATTATGACTTCGAAA GTTTATGATCCAGAACAAAG-3' | 17 |
| | 5′-(antisense)- CTAGTTGTTGTGTGGAATTTTATTGTTCATTT TTGAGAACTCGCTCAACGAAC-3′ | 18 |
| 2308 nt | 5′- CCATAATACGACTCACTATAGGGAGAAAAA TCGAGGGCCTCCCCTAGTTGTTGTGTGGAA TTATGGCCTTACCAGTGACCGC -3′ (sense) | 19 |
| | 5′ (antisense) CTAGTTGTTGTGTGGAATTTTACTTGTACAG CTCGTCCATGCCG-3′ | 20 |
| 3004 nt | 5′- CCATAATACGACTCACTATAGGGAGAAAAA TCGAGGGCCTCCCCTAGTTGTTGTGTGGAA TTATGTCTTATTCTGATGAGTCTCGACTGTC GAATC-3′ (sense) | 21 |
| | 5′- (antisense) CTAGTTGTTGTGTGGAATT TTACTTTTCAAACCTCCCTTCAGCCTGTTG- 3′ | 22 |
| 4000 nt | 5’- CCATAATACGACTCACTATAGGGAGAAAAA TCGAGGGCCTCCCCTAGTTGTTGTGTGGAA TTATGTCTTATTCTGATGAGTCTCGACTGTC GAATC-3′ (sense) | 23 |
| | | |
| | 5′- CTAGTTGTTGTGTGGAATTTTACTGAACTTC ACTGCACTGTGCC-3′ (antisense) | 24 |
| 5002 nt | 5′- CCATAATACGACTCACTATAGGGAGAAAAA TCGAGGGCCTCCCCTAGTTGTTGTGTGGAA TTATGTCTTATTCTGATGAGTCTCGACTGTC GAATC-3′ (sense) | 25 |
| | 5′- CTAGTTGTTGTGTGGAATT [?] TTACACAATCATGTCATCAGTGCTCTGTTCC -3′ (antisense) | 26 |

### 2. In vitro transcription (IVT) and purification

A circular RNA precursor was synthesized by performing *in vitro* transcription at 37°C for 3 hours using T7 RNA polymerase (200 U, Roche) from a linearized DNA plasmid or PCR amplification product under conditions of rATP, rCTP, rUTP, and rGTP (5 mM each, Roche), and rGMP (25 mM, Sigma), RNase inhibitor (0.2 U, Thermo Scientific), MgCl₂ (25 mM, Sigma), and DTT (10 mM, Thermo Scientific). After completion of transcription, a DNA template was removed by treating DNase I (4.5 U, Themo Scientific) at 37°C for 20 minutes. After DNase I treatment, RNA was column-purified using a Monarch RNA cleanup kit (New England Biolabs).

In some experiments, RppH (0.5 U, New England Biolabs) was treated for 30 minutes at 37°C after RNA purification.

### 3. RNA ligation by treatment of T4 RNA ligase 2

RNA was heat-treated at 80°C for 3 minutes, cooled to 26°C by 6°C per minute, and then treated with T4 RNA ligase 2 and reacted at 25°C for 1 hour. In addition, RNA was column-purified.

### 4. Splint ligation

In a 3'DB circular RNA precursor, a linear RNA having the same sequence except for the 19 nt-long sequence at the 3' end was designed, and a linear circular RNA precursor to be ligated was prepared using a DNA splint. Before ligation, 2 uM of RNA and 2 uM of the DNA splint were heat-treated at 80°C for 3 minutes and cooled to 26°C by 6°C per minute.

Three types of DNA splints of 30 nt, 60 nt, and 90 nt in length were used, and detailed information was shown in Table 2 below.

**[Table 2]**

| | | |
|---|---|---|
| DNA splint_30nt | 5'-CTCGATTTTTCTCCCGTCGACTTTTGTTTT-3' | SEQ ID NO: 27 |
| DNA splint_60nt | 5′- CAACTAGGGGAGACCCTCGATTTTCTCCCGTC GACTTTTGTTTTTTGTTTTTTTTTTTGT-3′ | SEQ ID NO: 28 |
| DNA splint_90nt | 5′- GTAATTCCACACAACAACTAGGGGAGACCCTC GATTTTTCTCCCGTCGACTTTT GTTTTTTGTTTTTTTTTTGTTTTTTGTTTTTTTTT TG-3′ | SEQ ID NO: 29 |

After ligation, DNase I (2.4 U, Themo Scientific) was treated at 37°C for 20 minutes to remove the DNA splint. Thereafter, RNA was column-purified.

### 5. Poly A tailing

After the ligation step, *E. coli* Poly (a) polymerase (0.5 U, New England Biolabs) and ATP (1 mM, New England Biolabs) were treated at 37°C for 1 hour, and the remaining RNA was column-purified.

### 6. RNase R treatment

RNA was heat-treated at 65°C for 5 minutes, cooled on ice for 3 minutes, and then treated with RNase R at 37°C for 15 minutes. Circular RNA was separated through column purification and quantified using Nanodrop 2000c (Thermo Scientific) spectrophotometers.

### 7. Electrophoresis

An RNA sample was mixed with a formamide loading buffer (Biosesang), denatured at 65°C for 10 minutes, and cooled on ice for 3 minutes. Then, RNA was transferred to a 1% agarose gel and separated at 100 V for 120 minutes under a MOPS buffer.

In some experiments, RNA was separated by preparing a 2% E-gel EX agarose gel (Invitrogen) using the E-gel EX 1-2% program in an E-gel Power Snap device. 1 kb (+) RNA ladder (Thermo Scientific) was used as a standard. Each band was visualized using the Azure 600 gel doc system (Azure Biosystems).

### [Experiment Results]

### 1. Confirmation of circularization of various dumbbell-structure circular RNA precursors using T4 RNA ligase 2

T4 RNA ligase 2 (Rnl2) was an enzyme for linking the 3'-end and 5'-end of double-stranded RNA or double-stranded RNA/DNA hybrid, and to prepare circular RNA using Rnl2, a DNA splint capable of complementarily binding to a part of both ends of a single-stranded RNA was used.

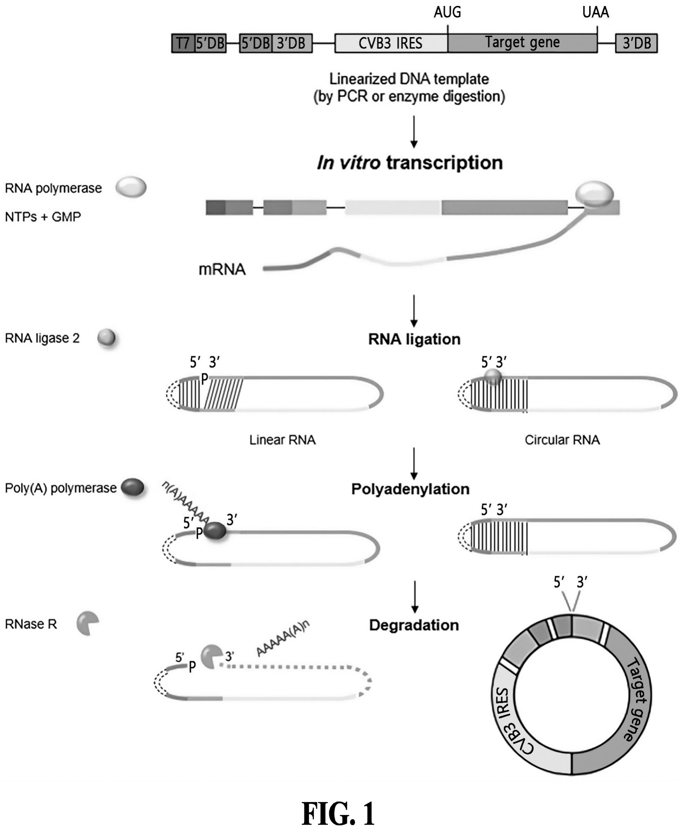

The present inventors designed and prepared a circular RNA structure that may be circularized by Rnl2 without a DNA splint by locating regions capable of complementarily binding to the ends of single-stranded RNA, as illustrated in FIG. 1.

The first prepared circular RNA precursor was designed to have a 13 bp-long 5' DB region and a 19 bp-long 3' DB region. The circular RNA precursor was named a wild type circular RNA precursor and was denoted as "DB-WT".

In addition, to confirm the presence or absence of the 5'DB region and 3'DB region and the effect of the lengths of the regions on ligation efficiency, a 3' dumbbell circular RNA precursor without a 5'DB region, a 5' dumbbell circular RNA precursor without a 3'DB region, and a 5' and 3' dumbbell deletion precursor without forming a dumbbell structure were designed and prepared. Each of the precursors was denoted by "3'DB", "5'DB", and "5'3'DB deletion".

After expressing the four types of RNA precursors *in vitro*, the 5' end was prepared with monophosphate by treatment with RppH, and then treated with T4 RNA ligase 2 (Rnl2) to induce ligation. Subsequently, by-products that were not ligated by Rnl2 were removed by treating poly (A) tailing and RNase R, and the sample was loaded on an agarose gel and subjected to electrophoresis to confirm the production of circular RNA.

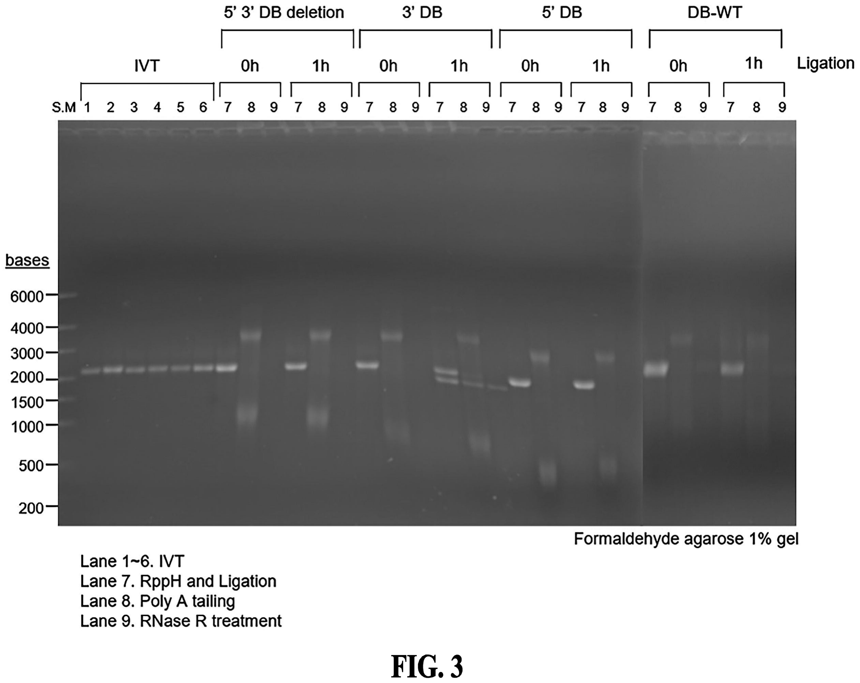

The results were illustrated in FIG. 3.

As can be seen in lane 9 of 5'3'DB deletion and 5'DB, all RNAs were removed by RNase R treatment despite Rnl2 treatment for 1 hour. In other words, it was confirmed that circular RNA was not formed by Rnl2 in 5'3'DB deletion and 5'DB.

In the case of 3'DB, no band was identified in lane 9 at 0 h of Rnl2 treatment, but a clear circular RNA band was identified in lane 9 after 1 h ligation induction, and in lane 7, two bands were identified due to the formation of circular RNA.

In the case of DB-WT, bands were confirmed in lane 9 both before (0 h) and after (1 h) ligation induction. The confirmation of the bands in lane 9 before ligation induction was shown as a result of not completely degrading linear RNA by RNase R due to the characteristics of the dumbbell structure of RNA. In addition, DB-WT did not function well with RppH due to its structural characteristics, resulting in low ligation efficiency, and a clear circular RNA band could not be identified in lane 7 after ligation induction.

### 2. Confirmation of ligation efficiency by shortening length of 3'DB region

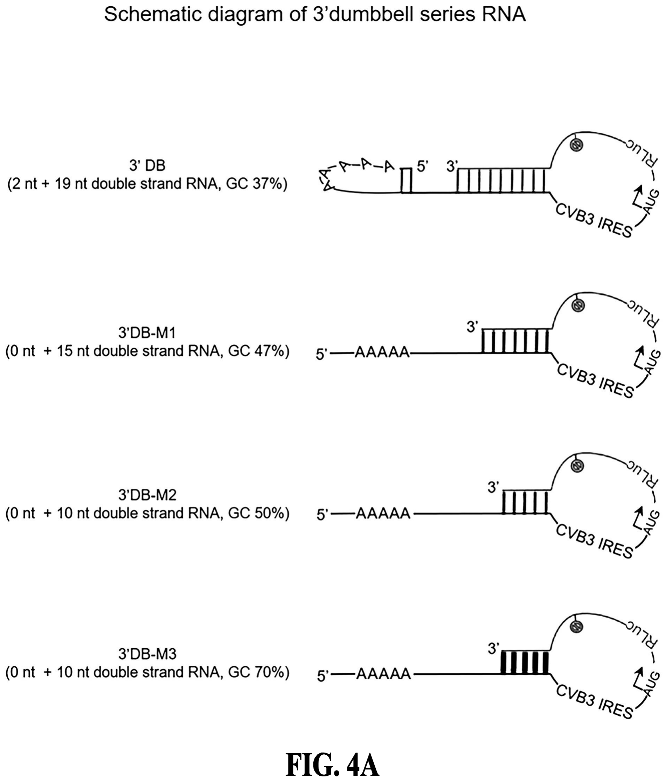

To improve the ligation efficiency by Rnl2, mutants of the 3'DB circular RNA precursor were designed and prepared by varying the length of the 3'DB region, the length of the 5' loop, and the GC content constituting the 3'DB region. The length of the 3'DB region, the length of the 5' loop, and the GC content constituting the 3'DB region of 3'DB and each mutant thereof were shown in Table 3 below, and a brief schematic diagram of the four types of circular RNA precursors was the same as FIG. 4A.

**[Table 3]**

| Circular RNA precursor | Length of 3'DB region | GC content of 3'DB region | Length of 5' loop |
|---|---|---|---|
| 3'DB | 19 bp | 37% | 21 nt |
| 3'DB-M1 | 15 bp | 47% | 10 nt |
| 3'DB-M2 | 10 bp | 50% | 10 nt |
| 3'DB-M3 | 10 bp | 70% | 10 nt |

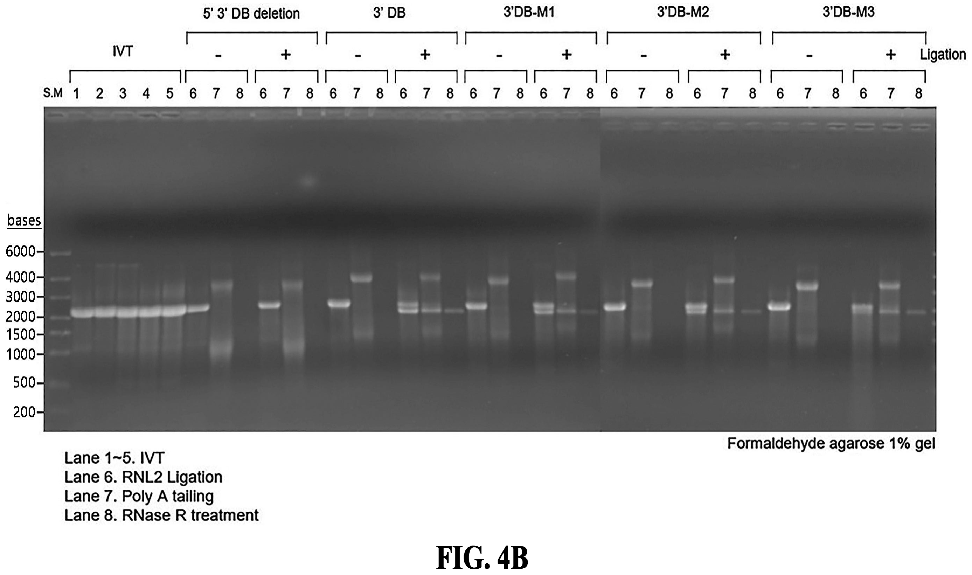

The three types of 3'DB circular RNA mutant precursors were expressed *in vitro* and then treated with T4 RNA ligase 2 (Rnl2) to induce ligation. Subsequently, by-products that were not ligated by Rnl2 were removed by treating poly (A) tailing and RNase R, and the sample was loaded on an agarose gel and subjected to electrophoresis to confirm the production of circular RNA (FIG. 4B). 5'3' DB deletion was used as a negative control, and 3' DB was used as a positive control.

In FIG. 4B, when ligation efficiency was confirmed from a band concentration in lane 6 in each circular RNA precursor, the ligation efficiencies were higher in the order of 3'DB, 3'DB-M1, 3'DB-M3, and 3'DB-M2. 3'DB-M3 produced higher circular RNA than 3'DB-M2, which was not shown as a significant value. From the results, in the ligation efficiency, it could be seen that the length of the 3'DB region was more important than the GC content of the 3'DB region.

### 3. Confirmation of ligation efficiency by shortening length of 5'DB region

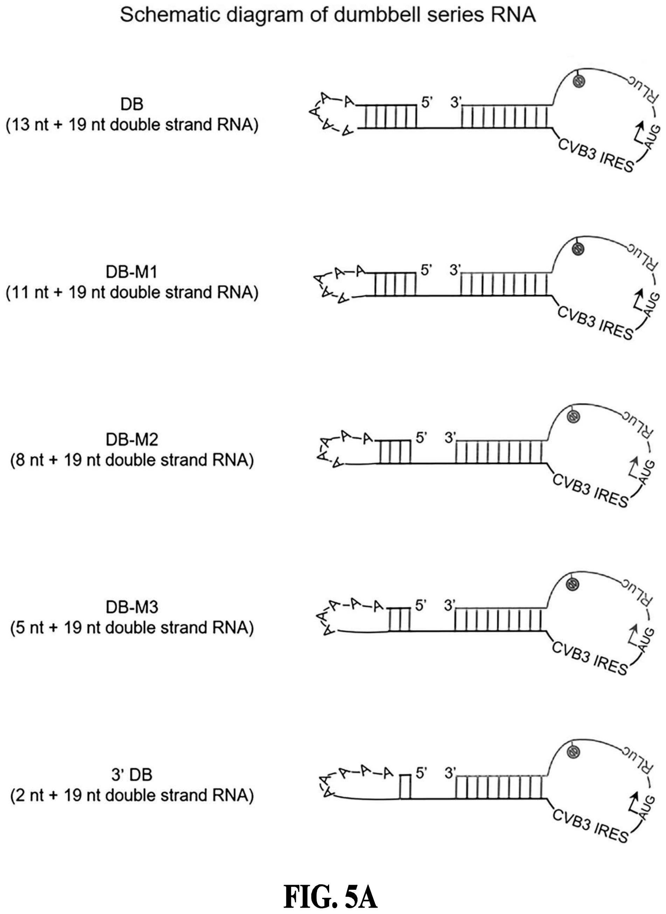

Meanwhile, dsRNA of 30 nt or longer within cells had a high risk of inducing cellular immune responses through activation of PKR. Accordingly, in order to use circular RNA as a gene therapy, the region forming a double strand is desirably less than 30 nt, and more desirably 30 nt or shorter. Therefore, the present inventors designed a DB circular RNA mutant precursor (DB-M) by shortening the length of the 5'DB region in DB-WT (FIG. 5A).

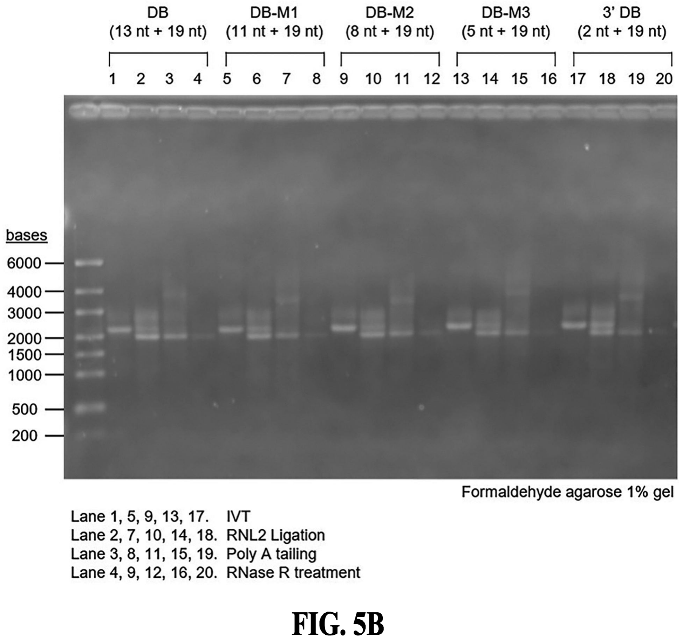

The DNA encoding the precursor was expressed *in vitro* and then treated with Rnl2 to induce ligation. Subsequently, by-products that were not ligated by Rnl2 were removed by treating poly (A) tailing and RNase R, and the sample was loaded on an agarose gel and subjected to electrophoresis to confirm the production of circular RNA (FIG. 5B). As a result, 3'DB had ligation efficiency of only 50% compared to DB-WT, but there was no significant difference in ligation efficiency between the three types of DB-M and DB-WT. From the results, it can be seen that DB-M3 is most suitable as a circular RNA precursor with the highest ligation efficiency even without inducing immune responses when introduced into cells.

### 4. Identification of maximum length of circular RNA

The present inventors designed DB-M3-based circular RNA precursors by varying the size of an inserted gene to determine whether circular RNA precursors may be used for expressing protein within cells, and confirmed circular RNA formation and the efficiency thereof by expressing the circular RNA precursors *in vitro,* and then treating Rnl2 and treated poly (A) tailing and RNase R, and performing electrophoresis on a formaldehyde agarose gel and EX 2% gel.

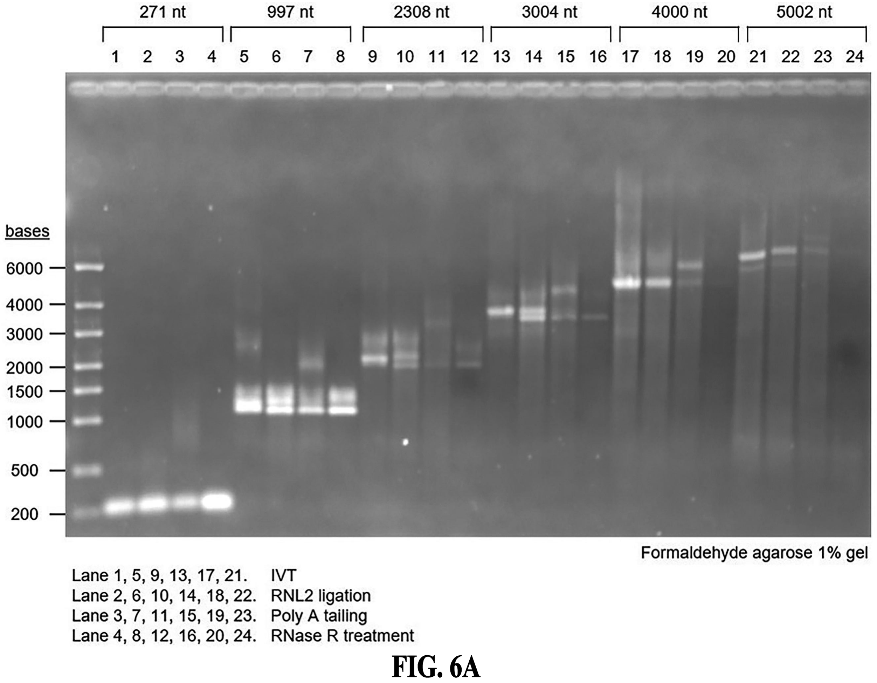

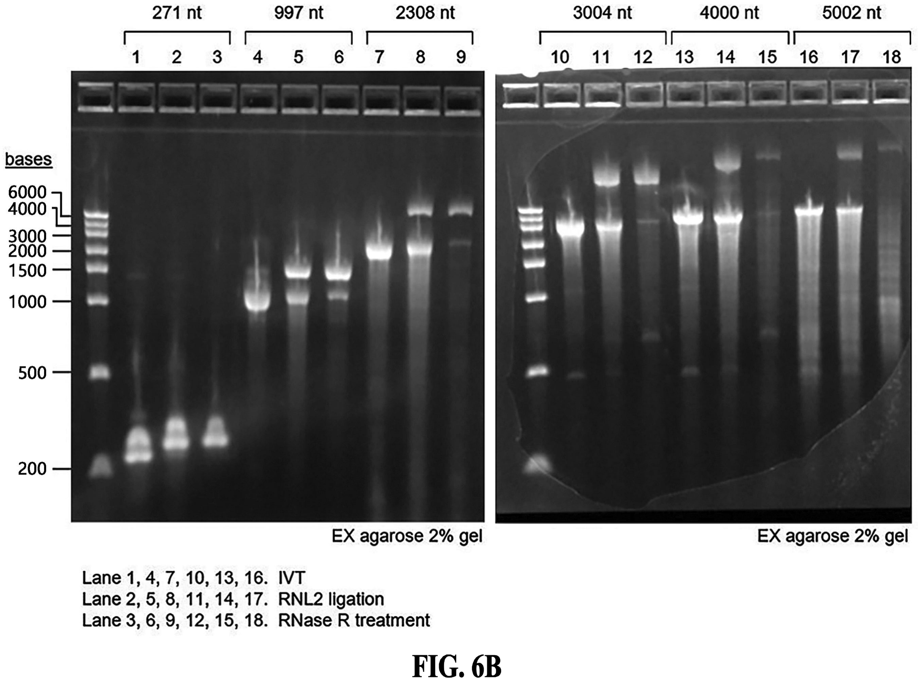

The results of electrophoresis on the formaldehyde agarose gel were shown in FIG. 6A, and the results of electrophoresis on the EX 2% agarose gel were shown in FIG. 6B.

In the case of the RNA precursor of 271 nt long, no double band was observed in the formaldehyde agarose gel, but a clear band was identified even upon RNase R treatment. In addition, bands that moved upward were identified in the EX agarose 2% agarose gel. From the results, it was found that the circular RNA precursor of 271 nt long formed circular RNA.

For RNA precursors with lengths of 997 nt, 2308 nt, and 3004 nt, clear double bands were observed on the formaldehyde agarose gel and bands moved upward were observed on the EX 2% agarose gel, and thus, it was confirmed that the circular RNA precursor was well ligated into circular RNA.

In the case of RNA precursors of 4000 nt and 5002 nt in length, clear double bands could not be confirmed on the formaldehyde agarose gel, but bands moved upward larger than the RNA size were clearly observed on the EX 2% agarose gel.

To confirm the ligation efficiency of the circular RNA precursors having each length, circular RNA bands identified on the EX 2% agarose gel were quantified and the ligation efficiency was calculated according to Equation 1 below. Ligation efficiency (%) = Circular band quantitative value/(Circular band quantitative value + Linear band quantitative value) × 100

As a result, the circular RNA precursor between 271 nt and 5002 nt was circularized by Rnl2 to produce circular RNA, but the ligation efficiency was higher the smaller the RNA size, but the circular RNA precursor of the present disclosure was able to insert genes up to about 5000 nt in length. Specifically, the ligation efficiency was about 90% for the 271-nt length and about 20% for the 5002-nt length.

### 5. Optimization of circular RNA preparation method

### 5-1. GTP : GMP ratio

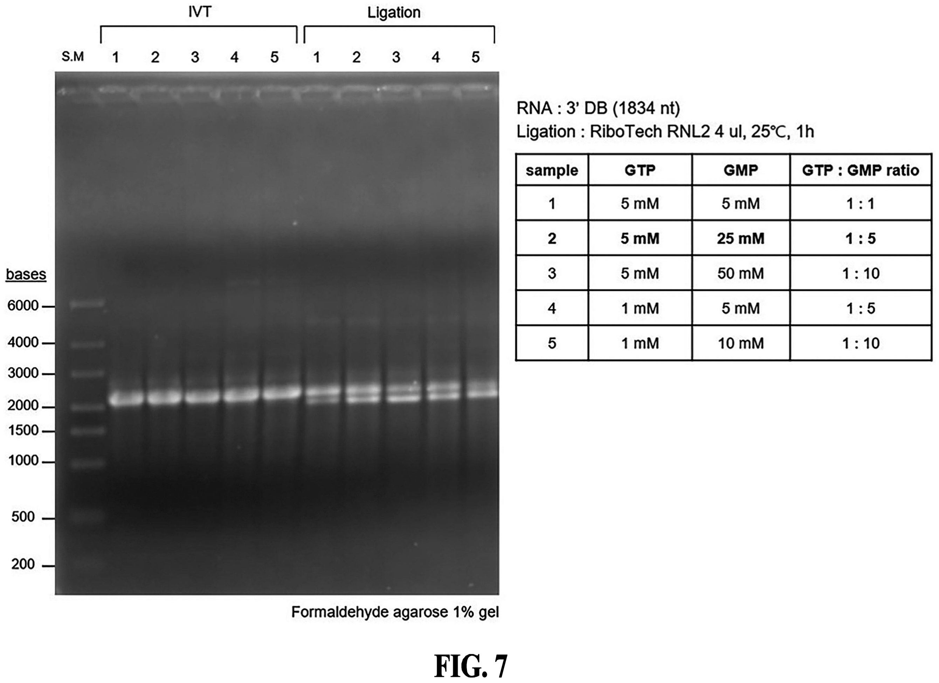

In order to ligate both ends of RNA by T4 RNA ligase 2, the 5' end needed to be monophosphate. Usually, to this end, RppH was treated. The present inventors induced *in vitro* transcription by adding GMP, and induced ligation by *in vitro* transcription of 3'DB by varying the ratio and amount of GTP and GMP and then confirmed the ligation efficiency (FIG. 7).

As a result, when GTP : GMP was 1 : 5, the ligation efficiency of about 50% was confirmed, and when the GMP ratio was higher therethan, circular RNA production was slightly increased. When comprehensively considering the total rNTP usage and ligation efficiency, it was confirmed that the GTP : GMP ratio was appropriately 1 : 5.

### 5-2. Heating condition

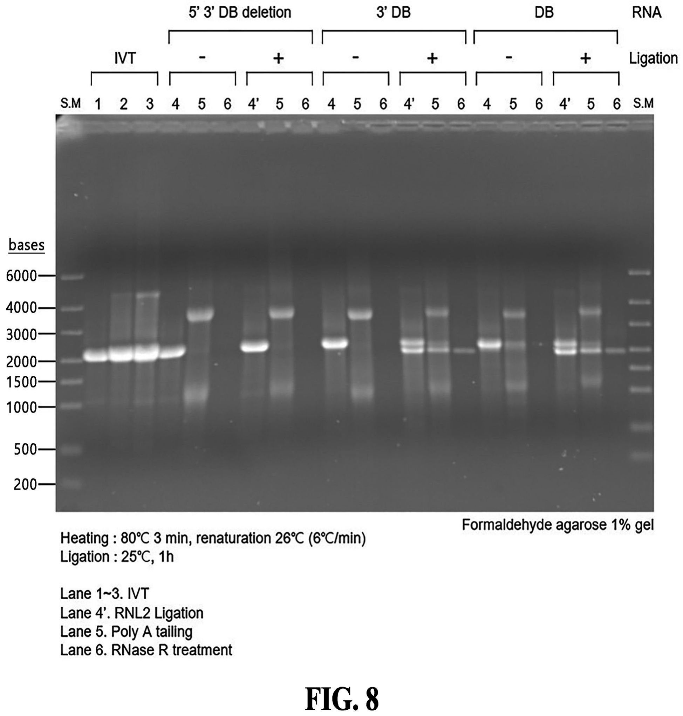

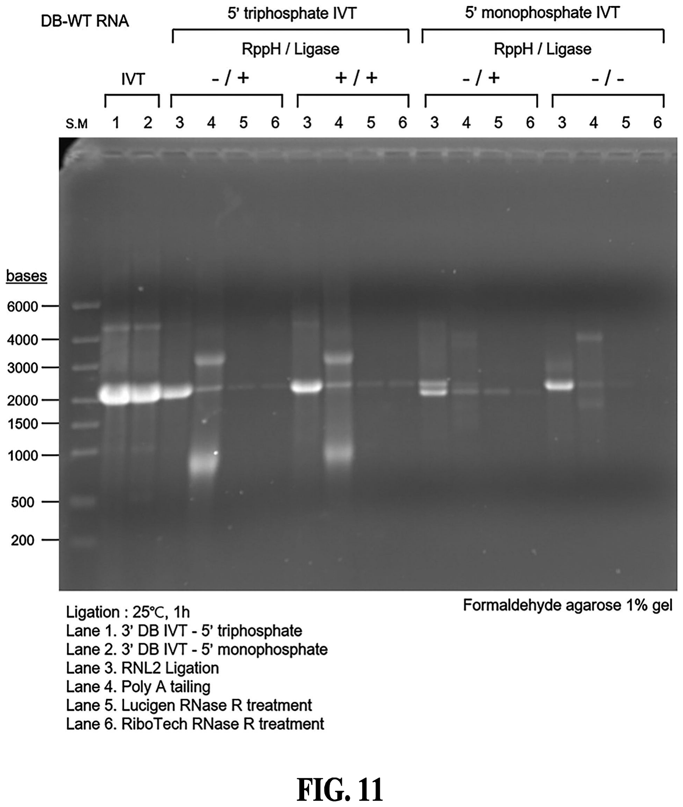

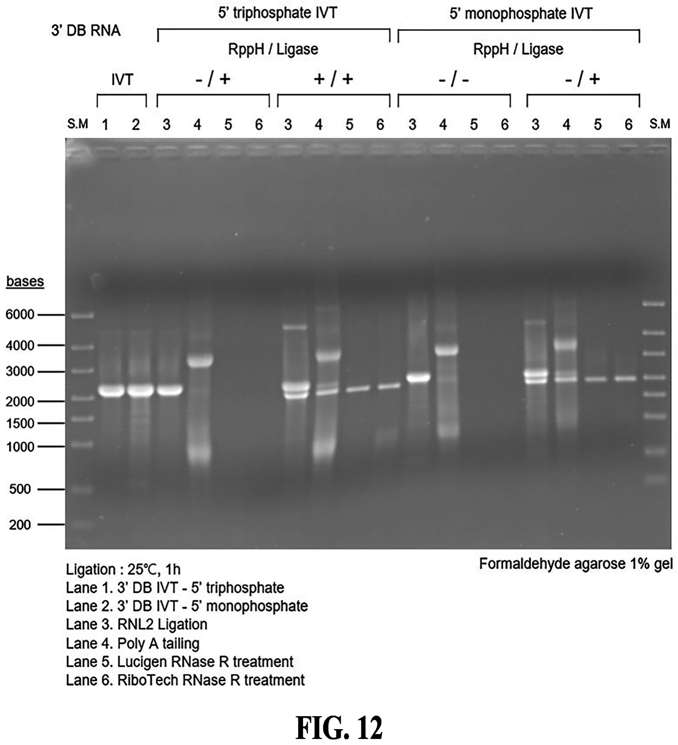

After *in vitro* transcription, a heating step was added before ligation to induce a transcript to form an intended secondary structure. Whether there was a difference in ligation efficiency depending on a condition of the heating step was confirmed. The ligation efficiency was compared in the case of heating treatment of heating at 65°C for 3 minutes and cooling on ice for 3 minutes, and in the case of heating treatment of cooling from 80°C to 26°C by 6°C per minute for 3 minutes. As a result, as illustrated in FIG. 8, it was confirmed that the heating treatment condition of cooling from 80°C to 26°C by 6°C per minute for 3 minutes had better ligation efficiency than the results in FIGS. 11 and 12, which were the former conditions.

### 5-3. Ligation reaction condition

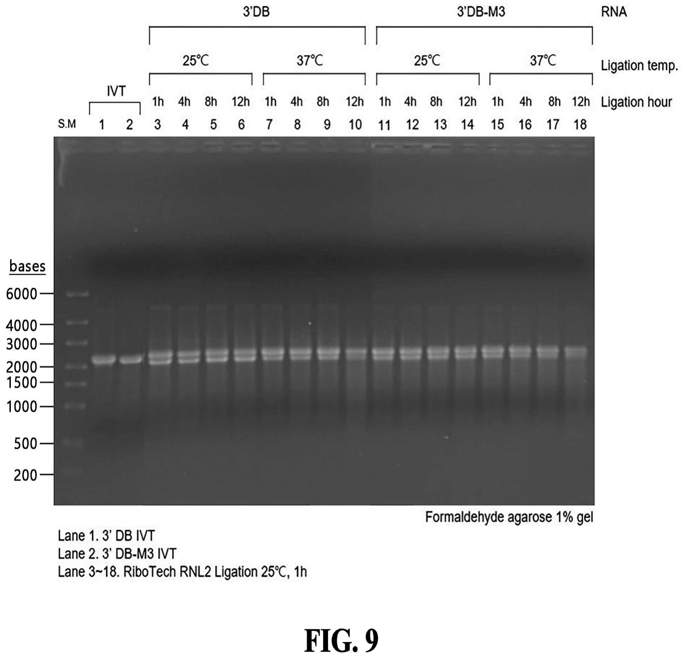

In the ligation reaction induction step, ligation efficiency was confirmed according to temperature and time conditions. Specifically, the ligation temperature condition was set to 25°C or 37°C, and the formation degree of circular RNA was confirmed through bands after 1, 4, 8, or 12 hours under the temperature conditions (FIG. 9). As a result, sufficient circular RNA bands were confirmed upon ligation induction at 25°C for 1 hour. As a result of inducing the reaction at 37°C, the circular RNA band was weak. Even if the ligation was induced for more than 1 h, no significant increase in ligation efficiency was confirmed.

### 5-4. RppH treatment

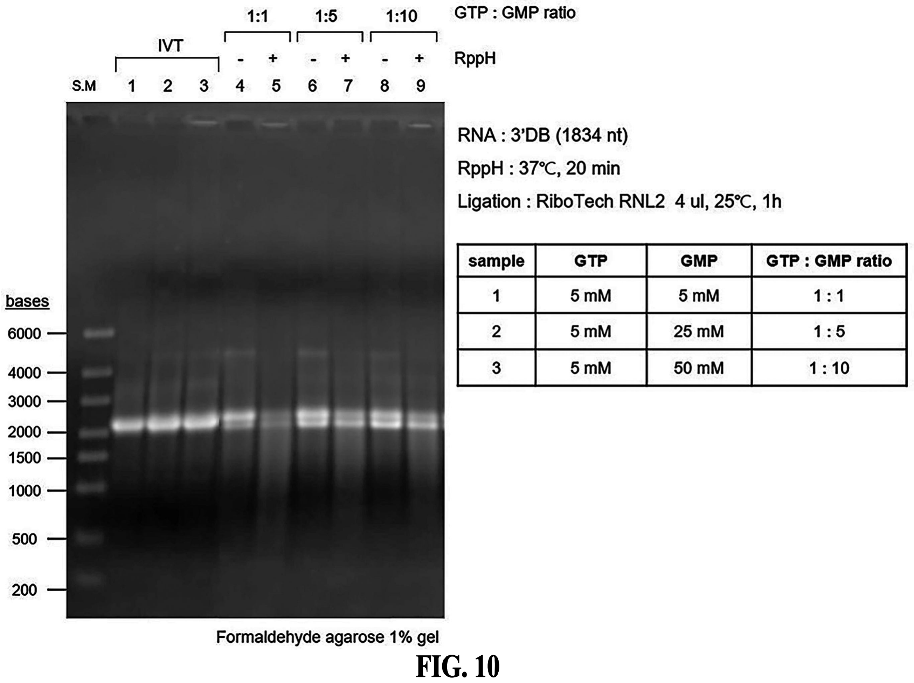

Meanwhile, in order to increase the efficiency of ligation induction by Rnl2, IVT was performed under various GTP : GMP conditions and an RppH treatment step was additionally performed to confirm the ligation efficiency. As a result, when the ligation induction by Rnl2 was performed after RppH treatment, the ligation efficiency was higher than that when RppH was not treated (FIG. 10).

### 5-5. Confirmation of circularization in DB-WT and 3'DB

Circular RNA was prepared under optimal circular RNA preparation conditions identified in 5-1 to 5-4 above using DB-WT and 3'DB circular RNA precursors circularized by Rnl2, and the efficiency thereof was confirmed. Two different circularization methods were compared by performing an IVT experiment under a condition of adding only GTP without GMP and then treating RppH to make the 5' end with monophosphate. The experiment result using DB-WT was illustrated in FIG. 11, and the experiment result using 3'DB was illustrated in FIG. 12.

As confirmed above, DB-WT and 3'DB were ligated by Rnl2 to produce circular RNA. Meanwhile, despite RppH treatment under the condition added with only GTP, DB-WT had circular RNA production efficiency lower than 3'DB. This is considered because in the case of DB-WT, RppH did not properly function due to its secondary structure. In addition, in the case of DB-WT, the linear RNA band was not completely removed in lane 6 after treatment of poly (A) tailing and RNase R due to a secondary structure, but in the case of 3'DB, linear RNA was not identified after the purification.

### 6. Confirmation of comparison of ligation efficiency with splint ligation

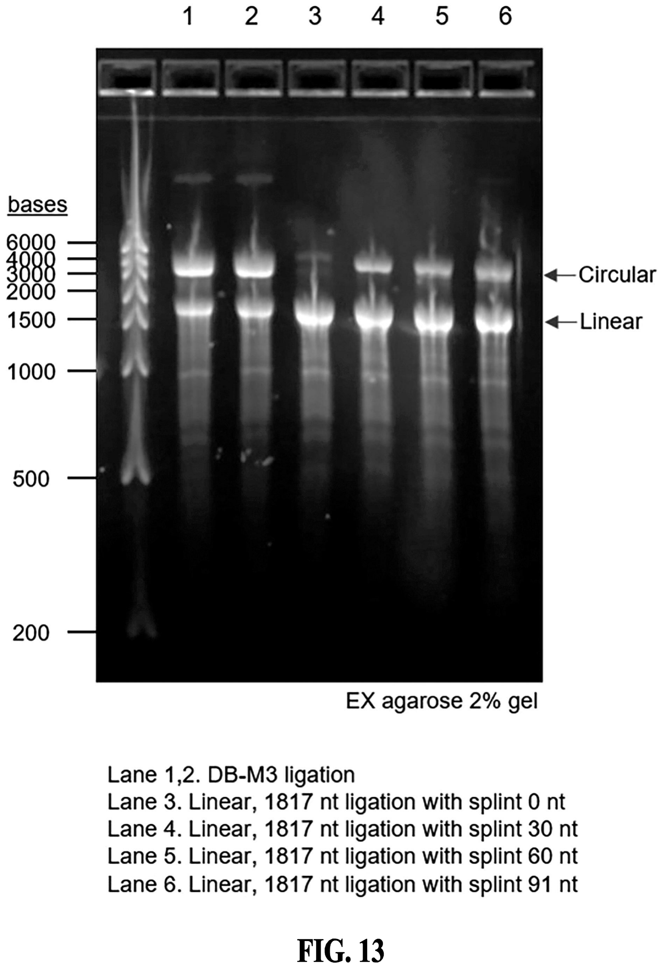

Circular RNA preparation efficiency was confirmed in the preparation of circular RNA using DB-M3 and the preparation of circular RNA using a DNA splint. The circularization condition was set to an optimal condition confirmed in 5-1 to 5-4 above, and in a circular RNA precursor used for splint ligation, a 19-nt nucleotide at the 3' end was deleted from DB-M3. A DNA splint of 30, 60, or 91 nt in length was used. As a result, as can be seen in FIG. 13, it was found that DB-M3 had better ligation efficiency by Rnl2 than when using three types of DNA splints.

As described above, although Examples have been described by the restricted drawings, various modifications and variations can be applied on the basis of Examples by those skilled in the art. For example, even if the described techniques are performed in a different order from the described method, and/or components such as a system, a structure, a device, a circuit, and the like described above are coupled or combined in a different form from the described method, or replaced or substituted by other components or equivalents, an appropriate result can be achieved.

Therefore, other implementations, other Examples, and equivalents to the appended claims fall within the scope of the claims to be described below.

### Industrial Applicability

The present disclosure can be used to prepare circular RNA.

## Claims

1. A circular RNA precursor, wherein the precursor has a structure of 5'-3'DumbBell Sense sequence (3'DB SS) - GOI - 3'DumbBell Antisense sequence (3'DB AS) - 3',
the 3'DB AS region consists of a reverse complementary sequence to the 3'DB SS region,
the 3'DB SS region has a length of 10 to 29 nt, and
the circular RNA precursor is circularized by Rnl2 without a DNA splint.

2. The circular RNA precursor of claim 1, wherein the precursor includes nucleotides extending in a 5' direction of the 3'DB SS region,
the region consisting of the extending nucleotides includes a structure of 5'DumbBell Antisense sequence (5'DB AS) - 5'DumbBell Sense sequence (5'DB SS),
the 5'DB AS region consists of a reverse complementary sequence to the 5'DB SS region, and
the 5'DB SS region has a length of 3 to 15 nt.

3. The circular RNA precursor of claim 2, wherein the 5'DB AS region and the 5'DB SS region are linked by 1 to 19 random nucleotides.

4. The circular RNA precursor of claim 1, wherein a length of the GOI region is 500 to 5500 nt.

5. The circular RNA precursor of claim 1, wherein the GOI includes an IRES.

6. An expression vector capable of expressing the circular RNA precursor of any one of claims 1 to 5.

7. The expression vector of claim 6, further comprising:
a promoter operably linked to a gene encoding the circular RNA precursor.

8. A method for preparing circular RNA comprising:
(1) inducing *in vitro* transcription of the expression vector of claim 6 under a condition of GTP : GMP = 1 : 5 to 10;
(2) treating DNase I *in vitro* after the transcription induction is completed; and
(3) inducing ligation by adding Rnl2 *in vitro.*

9. The method for preparing circular RNA of claim 8, further comprising:
treating RppH *in vitro* after the 2 is performed.

10. The method for preparing circular RNA of claim 8, further comprising:
heating immediately before the 3,
wherein the heating is performed by heating at 80°C for 3 minutes and cooling to 26°C by 6°C per minute.

11. The method for preparing circular RNA of claim 8, wherein the 3 is performed at 25°C for 1 h or more.

12. The method for preparing circular RNA of claim 8, further comprising: (4) treating RNase R *in vitro* after the 3.

13. The method for preparing circular RNA of claim 12, further comprising:
treating poly (A) polymerase *in vitro* immediately before the 4.
